(19) Europäisches Patentamt
European Patent Office
Office européen des brevets

(11) Veröffentlichungsnummer: **0 264 844 B1**

(12) **EUROPÄISCHE PATENTSCHRIFT**

(45) Veröffentlichungstag der Patentschrift: **26.08.92**

(51) Int. Cl.⁵: **A61K 7/48**, A61K 7/06

(21) Anmeldenummer: **87115137.9**

(22) Anmeldetag: **16.10.87**

# (54) Kosmetische Emulsionen mit verbessertem Fliessverhalten.

(30) Priorität: **24.10.86 DE 3636256**

(43) Veröffentlichungstag der Anmeldung:
**27.04.88 Patentblatt 88/17**

(45) Bekanntmachung des Hinweises auf die Patenterteilung:
**26.08.92 Patentblatt 92/35**

(84) Benannte Vertragsstaaten:
**AT BE CH DE ES FR GB IT LI NL SE**

(56) Entgegenhaltungen:
**EP-A- 0 058 853**
**DE-C- 837 997**
**US-A- 4 009 254**

**CHEMICAL ABSTRACTS, Band 79, Nr. 16, 22. Oktober 1973, Seite 317, Zusammenfassung Nr. 96851h, Columbus, Ohio, US**

**CHEMICAL ABSTRACTS, Band 79, Nr. 10, 10. September 1973, Seite 288, Zusammenfassung Nr. 57575b, Columbus, Ohio, US**

**PATENT ABSTRACTS OF JAPAN, Band 7, Nr. 181 (C-180)[1326], 10. August 1983**

**Englische Übersetzung von JP 73/33037 und JP 73/5941**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Höffkes, Horst, Dr.**
**Carlo-Schmid-Strasse 113**
**W-4000 Düsseldorf(DE)**
Erfinder: **Lange, Fritz, Dr.**
**Baderweg 82**
**W-4300 Essen(DE)**
Erfinder: **Ansmann, Achim, Dr.**
**Fichtestrasse 19**
**W-4010 Hilden(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft kosmetische Emulsionen vom Typ Öl-in-Wasser, die bei Normaltemperatur fließfähig sind und die eine hohe Stabilität und ein verbessertes Fließverhalten aufweisen.

Viskosität und rheologisches Verhalten von Öl-in-Wasser-Emulsionen haben großen Einfluß auf die Stabilität und auf die anwendungstechnischen Eigenschaften von Emulsionen. So ist z. B. bekannt, daß sich die Stabilität von Emulsionen erhöht, wenn die Viskosität erhöht wird. Kosmetische Cremes enthalten daher oft konsistenzgebende Komponenten, wie z. B. Fettalkohole, Fettsäurepartialglyceride oder Wachse, die einen wesentlichen Beitrag zur Stabilität der Emulsion leisten. Es gibt aber zahlreiche Emulsionen, bei welchen aus anwendungstechnischen Gründen eine flüssige oder gießfähige Konsistenz erwünscht ist. Man kann ein Viskositätserniedrigung z. B. durch eine Verringerung der inneren Phase oder durch Ersatz der konsistenzgebenden Komponenten durch flüssigere Komponenten erreichen. Eine Verringerung der inneren Phase ist aber anwendungstechnisch meist unerwünscht, da sie den Gehalt und das Aussehen der Emulsion verschlechtert, d. h. die Emulsionen sehen dann durchscheinend und ästhetisch wenig ansprechend aus. Ein Weglassen oder Verringern der konsistenzgebenden Komponenten ist oft mit einem Verlust der Emulsionsstabilität verbunden.

In Emulsionen mit kationischen Emulgatoren, in welchen Fettalkohole und Fettsäurepartialglyceride als konsistenzgebende Komponenten enthalten sind und die als flüssige Haarnachbehandlungsmittel gebräuchlich sind, besteht das Problem, daß Fettalkohole wenig zu einem cremigen, gehaltvollen Aussehen solcher Emulsionen beitragen und daß Fettsäurepartialglyceride die Avivagewirkung eher verringern. Außerdem neigen solche Emulsionen zum Nachdicken, d. h. es besteht die Gefahr, daß sich nach längerer Lagerzeit die Konsistenz wesentlich erhöht oder daß die Fließfähigkeit völlig verlorengeht.

Die amerikanische Patentschrift US 4 009 254 beschreibt kosmetische Emulsionen, welche Dialkylether der Formel $R_1$ - O - $R_2$ enthalten, wobei $R_1$ einen $C_8$ - $C_{20}$ - Alkylrest und $R_2$ einen kurzkettigen, gesättigten oder ungesättigten Rest mit 1 bis 3 Kohlenstoffatomen darstellt. Dieser kurzkettige Rest ist für die niedrige Viskosität der Verbindungen verantwortlich.

Die japanischen Patentanmeldungen JP 73/33037 und JP 73/5941 offenbaren kosmetische Formulierungen, die Dialkylether in hohen Mengen, beispielsweise zwischen 15 und 90 Gew.-% bzw. zwischen 12 und 35 Gew.-%, als Ersatz für bekannte Ölkomponenten wie natürliches Sebum enthalten.

Aus der europäischen Patentanmeldung EP 58 854 und der japanischen Patentanmeldung JP 83/85 810 ist bekannt, daß quartäre Ammoniumsalze als Emulgatoren eingesetzt werden können.

Es wurde nun gefunden, daß man fließfähige Emulsionen vom Typ Öl-in-Wasser (O/W) mit erniedrigter Viskosität und hoher Lagerstabilität erhält, wenn man als Ölkomponente Dialkylether mit Alkylgruppen mit 6 bis 22 C-Atomen verwendet. Gegenstand der Erfindung sind daher kosmetische Emulsionen vom Typ Öl-in-Wasser, die bei 20 °C fließfähig sind und die dadurch gekennzeichnet sind, daß sie 1 bis 30 Gew.-% einer diskontinuierlichen lipophilen und 70 bis 99 Gew.-% einer kontinuierlichen wäßrigen Phase sowie als Ölkomponente 0,5 bis 5 Gew.-% Dialkylether der Formel $R^1$ - O - $R^2$, in welcher $R^1$ und $R^2$ unabhängig voneinander lineare oder verzweigte Alkylgruppen mit 6 bis 22 C-Atomen sind, neben üblichen Öl- und Fettkomponenten enthalten.

Dialkylether der Formel $R^1$ - O - $R^2$ sind literaturbekannt, solche Dialkylether, die nicht bereits literaturbekannt sind, lassen sich nach allgemein bekannten Verfahren leicht z. B. aus Alkoholen durch Wasserabspaltung oder durch Alkylierung von Alkoholen mit Alkylhalogeniden oder Alkylsulfaten herstellen. Die Herstellung symmetrischer Dialkylether wird z. B. von R. Perron und Ch. Paquot in Compt. rend. 231, 237 - 238 (1950) beschrieben. Die Herstellung unsymmetrischer Dialkylether durch Alkylierung von Alkoholen mit Alkylschwefelsäuremonoester-Salzen wird in einem Beispiel näher beschrieben.

Für die Herstellung der erfindungsgemäßen O/W-Emulsionen können als Ölkomponenten auch Mischungen von Dialkylethern der Formel $R^1$ - O - $R^2$ und üblichen kosmetischen Öl- und Fettkomponenten eingesetzt werden. Bereits ein Gehalt von 0,5 bis 5 Gew.-% an Dialkylethern neben üblichen Öl- und Fettkomponenten, bezogen auf die gesamte Emulsion, kann die Viskosität der Emulsion merklich senken. Es ist daher auch ohne Belang, wenn die verwendeten Dialkylether nicht in reiner Form, sondern z. B. in Form eines Gemisches mit den für ihre Herstellung verwendeten Ausgangsalkoholen eingesetzt werden.

Die viskositätserniedrigende Wirkung der Dialkylether ist in O/W-Emulsionen generell feststellbar, jedoch von besonderem praktischem Wert bei solchen Emulsionen, die bei 20 °C fließfähig sein sollen und einen Gehalt von 1 bis 30 Gew.-% diskontinuierlicher lipophiler und 70 bis 99 Gew.-% kontinuierlicher (äußerer) wäßriger Phase aufweisen.

Die lipophile Phase besteht im wesentlichen aus kosmetischen oder pharmazeutischen Ölkomponenten, Fetten und/oder Wachsen, öllöslichen Emulgatoren und gegebenenfalls öllöslichen pharmazeutischen oder kosmetischen Wirkstoffen. Als kosmetische Ölkomponenten können neben den erfindungsgemäß enthaltenen Dialkylethern alle hierfür bekannten pflanzlichen, tierischen, mineralischen und synthetischen Öle enthalten sein. Als Beispiele seien genannt: Olivenöl, Sonnenblumenöl, Maiskeimöl, Spermöl, Nerzöl, Paraffinöl, Silikonäle (z. B. Dimethylpolysiloxan), Squalan, Oleylalkohol, 2-Octyl-dodecanol, Decyloleat, Isopropylmyristat, Isononylstearat, 2-Ethylhexyl-palmitat, Glycerin-tricaprylat und andere alskosmetische Ölkomponenten bekannte Ester, Alkohole oder Kohlenwasserstoffe. Als kosmetische Fette und Wachse eignen sich alle hierfür bekannten Produkte mit Schmelzpunkten bis ca. 80 °C. Als Beispiele seien genannt gehärtete pflanzliche und tierische Fette (Triglyceride), Walrat, Fettalkohole, z. B. Cetylalkohol, Stearylalkohol, Ester wie z. B. Cetylpalmitat und natürliche Wachse wie z. B. Wollwachs, Bienenwachs, Japanwachs, Carnaubawachs, Candelillawachs, mineralische Wachse wie z. B Montanwachs, Paraffine, Vaseline sowie synthetische Paraffine wie z. B. die Polyethylenwachse.

Als öllösliche Emulgatoren eignen sich alle für die Emulgierung der vorgenannten Öle, Fette und Wachse geeigneten, öllöslichen Emulgatoren. Als Beispiele seien genannt die Seifen von Fettsäuren mit 12 bis 22 C-Atomen, die Mono- und Diglyceride und die Sorbitan-Partialester von Fettsäuren mit 12 bis 22 C-Atomen und die Anlagerungsprodukte von 2 bis 30 Mol Ethylenoxid an solche Fettsäure-Partialglyceride und Sorbitanfettsäureester, die Anlagerungsprodukte von 2 bis 30 Mol Ethylenoxid an Fettalkohole mit 12 bis 22 C-Atomen, an Fettsäuren mit 12 bis 22 C-Atomen, an Alkylphenole mit 8 bis 16 C-Atomen in der Alkylgruppe und an Fettsäurealkanolamide, die Fettalkoholsulfate mit 16 bis 22 C-Atomen in Form ihrer Alkali- oder Alkanolammoniumsalze, die Phosphorsäureester linearer Fettalkohole oder von Fettalkoholpolyglycolethern in Form der Alkali- oder Alkanolammoniumsalze.

Eine bevorzugte Ausführungsform der Erfindung sind solche Emulsionen vom Typ O/W, die als Emulgatoren oberflächenaktive quartäre Ammoniumsalze enthalten. Bevorzugt geeignet sind quartäre Ammoniumsalze der Formel

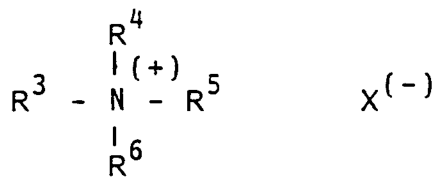

in der $R^3$ eine Alkylgruppe mit 12 bis 22 C-Atomen, $R^4$ eine Alkylgruppe mit 1 bis 4 C-Atomen, eine Benzylgruppe oder eine Gruppe $R^3$ ist und $R^5$ und $R^6$ Alkylgruppen mit 1 bis 4 C-Atomen oder Hydroxyalkylgruppen mit 2 bis 4 C-Atomen und $X^{(-)}$ ein Anion aus der Gruppe Chlorid, Bromid und $R^7 - O - SO_3^{(-)}$, worin $R^7$ eine $C_1$-$C_4$-Alkylgruppe ist, darstellt. Diese kationischen Emulgatoren werden bevorzugt in einer Menge von 0,2 bis 2 Gew.-% der Emulsion eingesetzt.

Beispiele für geeignete quartäre Ammoniumsalze sind z. B. Cetyltrimethylammoniumchlorid, Stearyltrimethylammoniumchlorid und Distearyl-dimethylammoniumchlorid.

Neben den genannten Bestandteilen kann die diskontinuierliche lipophile Phase noch öllösliche Wirkstoffe wie z. B. Lichtschutzmittel, Antioxidantien, Vitamine, öllösliche Konservierungsmittel, z. B. p-Hydroxybenzoesäurebenzylester oder pharmazeutische Wirkstoffe enthalten.

Die kontinuierliche wäßrige Phase kann neben dem Wasser noch weitere wasserlösliche Hilfsmittel enthalten. Als solche können z. B. Polyole wie Glycerin oder Sorbit, wasserlösliche Salze, z. B. Magnesiumsulfat, Puffersubstanzen, z. B. Alkaliphosphat, Alkalicitrat, Borate, wasserlösliche Konservierungsmittel wie z. B. p-Hydroxybenzoesäuremethylester, Sorbinsäure, wasserlösliche Polymere wie z. B. Carboxymethylcellulosen, Carobxyvinylpolymerisate, wasserlösliche oberflächenaktive Stoffe oder Emulgatoren, wasserlösliche Farbstoffe oder wasserlösliche kosmetische oder pharmazeutische Wirkstoffe, z. B. wasserlösliche Pflanzenextrakte, wasserlösliche Proteine oder Proteinderivate, Aminosäuren usw. genannt werden.

Die erfindungsgemäßen O/W-Emulsionen zeichnen sich durch eine erniedrigte Viskosität aus und weisen trotzdem eine hohe Stabilität und ein ansprechendes, cremiges Aussehen auf, so daß die Emulsionen auch in geringer Schichtdicke nicht durchscheinend wirken. Die Neigung zum Nachdicken, d. h. zum Viskositätsanstieg bei längerer Lagerung, ist deutlich verringert. Mit erfindungsgemäßen, kationischen O/W-Emulsionen, die als Haarnachbehandlungsmittel dienen, wird eine gute Haaravivagewirkung erzielt. Solche kationischen Emulsionen verleihen aber auch der Haut ein weiches, samtiges Gefühl.

**Beispiele**

1. Herstellung von n-Octyl-cetyl/stearylether

2 604 g (20 Mol) n-Octanol-1 und 360,6 g einer 30 Gew.-%igen Lösung von Natriummethylat in Methanol wurden zusammengegeben und durch Erhitzen auf 150 °C (unter $N_2$-Atmosphäre) Methanol abdestilliert. Dann wurden portionsweise 686 g (2 Mol) Cetyl-/stearyl(1:1)-sulfat-Natriumsalz (pulverförmig, 90%ig) zugegeben und das Reaktionsgemisch auf 175 °C erwärmt und 8 Stunden bei dieser Temperatur gerührt. Nach dem Abkühlen auf 90 °C wurde das Reaktionsgemisch mit 1 l Wasser vermischt. Die organische Phase wurde abgetrennt und noch zweimal mit Wasser gewaschen. Es wurden 758 g einer festen, farblosen Substanz mit einer Rest-Hydroxylzahl von 29,1 erhalten.

2. Herstellung von n-Octadecyl-cetyl/stearylether

Unter Stickstoff-Atmosphäre wurden 39 kg n-Octadecanol (144 Mol) und 8,6 kg 30%ige Natriummethylatlösung vorgelegt und auf 150 °C erwärmt, wobei Methanol abdestillierte. Nach einer Stunde wurden bei dieser Temperatur 10,4 kg Cetyl-/stearyl(1:1)sulfat-Natriumsalz (pulverförmig, 90%ig) zugegeben und eine Stunde bei 175 °C gerührt. Der gebildete Ether diente als zusätzliches Lösungsmittel. Weitere 10,4 kg Cetyl-/stearyl(1:1)-sulfat wurden zugefügt und 8 Stunden bei 175 °C gerührt. Nach Abkühlen auf 100 °C wurden 40 kg n-Hexanol zugegeben und zweimal mit Wasser gewaschen. Das Produkt wurde durch Destillation von n-Hexanol und überschüssigem n-Octadecanol befreit. Man erhielt 23,4 kg eines wachsartigen Produktes mit einer Rest-Hydroxylzahl von 31.

3. Haarspülmittel

| | 1.1 | 1.2 | 1.3 |
|---|---|---|---|
| Di-n-dodecylether | - | 1,5 | - |
| Cetyl/stearylalkohol | 3 | 1,5 | 1,5 |
| Glycerin-mono/di-palmitat/stearat | - | - | 1,5 |
| Cetyltrimethylammoniumchlorid (30%ige Lösung) | 2,0 | 2,0 | 2,0 |
| Wasser | 95 | 95 | 95 |
| Aussehen | opak transparent | weiß cremig | weiß cremig |
| Viskosität mPa s , 20 °C Brookfield RVF, Spindel 4, 20 UPM | | | |
| nach 24 Stunden | 1525 | 725 | 1650 |
| nach 30 Tagen | 2800 | 750 | 2500 |
| Avivagewirkung | sehr gut | sehr gut | gut |

4. Hautpflege-Lotionen

| | 2.1 | 2.2 | 2.3 | 2.4 |
|---|---|---|---|---|
| Di-n-dodecylether | - | 0,5 | 1,0 | - |
| n-Octyl-cetyl/stearylether | - | - | - | 2,0 |
| Paraffinöl (perliquidum) | 4,0 | 4,0 | 4,0 | 5,0 |
| Isopropylpalmitat | 8,0 | 8,0 | 8,0 | 3,0 |
| Silikonöl (M 300) | - | - | - | 1,0 |
| Cetyl/stearylalkohol | 1,0 | 0,5 | - | - |
| Cetyl/stearylalkohol-poly(12)-glycolether | 1,5 | 1,5 | 1,5 | 0,3 |
| Distearyl-dimethyl-ammonium-chlorid (70%ige Lösung) | - | - | - | 0,5 |
| Stearinsäure | 1,0 | 1,0 | 1,0 | - |
| Triethanolamin | 0,6 | 0,6 | 0,6 | - |
| p-Hydroxybenzoesäure-methyl ester | 0,1 | 0,1 | 0,1 | 0,2 |
| p-Hydroxybenzoesäure-propyl ester | 0,1 | 0,1 | 0,1 | 0,1 |
| Carbopol(R) 940 | 0,2 | 0,2 | 0,2 | - |
| Glycerin | 4,0 | 4,0 | 4,0 | 3,0 |
| Parfümöl | - | - | - | 0,3 |
| Wasser | 79,5 | 79,5 | 79,5 | 84,6 |
| Viskosität mPa s , 20 °C Brookfield RVf, Spindel 5, 10 UPM | | | | |
| nach 1 Stunde | 29200 | 17200 | 15400 | |
| nach 24 Stunden | 32800 | 23600 | 17000 | |

**Patentansprüche**

1. Kosmetische Emulsionen vom Typ Öl-in-Wasser, die bei 20 °C fließfähig sind, dadurch gekennzeichnet, daß sie 1 bis 30 Gew.-% einer diskontinuierlichen lipophilen und 70 bis 99 Gew.-% einer kontinuierlichen wäßrigen Phase sowie als Ölkomponente 0,5 bis 5 Gew.-% Dialkylether der Formel $R^1$-O-$R^2$, in welcher $R^1$ und $R^2$ unabhängig voneinander lineare oder einfach verzweigte Alkylgruppen mit 6 bis 22 C-Atomen sind, neben üblichen Öl- und Fettkomponenten enthalten.

2. Kosmetische Emulsionen nach Anspruch 1, dadurch gekennzeichnet, daß als Emulgatoren oberflächenaktive quartäre Ammoniumsalze enthalten sind.

**3.** Kosmetische Emulsionen nach Anspruch 2, dadurch gekennzeichnet, daß als Emulgatoren quartäre Ammoniumsalze der Formel,

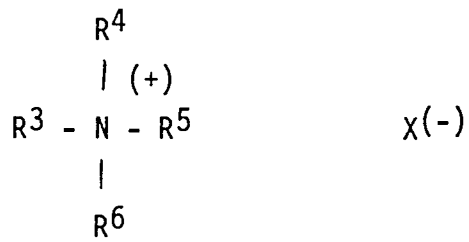

in der $R^3$ eine Alkylgruppe mit 12 bis 22 C-Atomen, $R^4$ eine Alkylgruppe mit 1 bis 4 C-Atomen, eine Benzylgruppe oder eine Gruppe $R^3$ ist und $R^5$ und $R^6$ Alkylgruppen mit 1 bis 4 C-Atomen und $X^{(-)}$ ein Anion aus der Gruppe Chlorid, Bromid und $R^7\text{-}OSO_3^{(-)}$, worin $R^7$ eine $C_1\text{-}C_4$-Alkylgruppe ist, darstellen, enthalten sind.

**4.** Verwendung von kosmetischen Emulsionen nach den Ansprüchen 1 bis 3 zur Pflege der Haut und/oder der Haare.

## Claims

**1.** Cosmetic emulsions of the oil-in-water type which are pourable at 20 °C, characterized in that they contain 1 to 30% by weight of a discontinuous lipophilic phase and 70 to 99% by weight of a continuous aqueous phase and, as oil component, 0.5 to 5% by weight dialkyl ethers corresponding to the formula $R^1$ - O - $R^2$, in which $R^1$ and $R^2$ independently of one another represent linear or branched $C_{6-22}$ alkyl groups in addition to typical oil and fatty components.

**2.** Cosmetic emulsions as claimed in claim 1, characterized in that they contain surface-active quaternary ammonium salts as emulsifiers.

**3.** Cosmetic emulsions as claimed in claim 2, characterized in that they contain as emulsifiers quaternary ammonium salts corresponding to the following formula

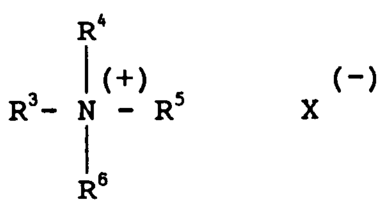

in which $R^3$ is a $C_{12-22}$ alkyl group, $R^4$ is a $C_{1-4}$ alkyl group, a benzyl group or a group $R^3$ and $R^5$ and $R^6$ are $C_{1-4}$ alkyl groups and $X^{(-)}$ is an anion from the group comprising chloride, bromide and $R^7$ - O - $SO^{(-)}$, where $R^7$ is a $C_{1-4}$ alkyl group.

**4.** The use of the cosmetic emulsions claimed in claims 1 to 3 for skin care and/or hair care.

## Revendications

**1.** Emulsions cosmétiques du type huile dans l'eau, qui sont coulantes à 20 °C et qui sont caractérisées en ce qu'elles renferment 1 à 30 % en poids d'une phase discontinue lipophile et 70 à 99 % en poids d'une phase continue aqueuse, de même que, en tant que composant huileux, 0,5 % à 5 % en poids de dialkyléthers de formule $R^1$ - O - $R^2$, dans laquelle $R^1$ et $R^2$ sont indépendamment l'un de l'autre des groupements alkyle linéaires ou simplement ramifiés comportant 6 à 22 atomes de C, plus des composants huileux et gras usuels.

**2.** Emulsions cosmétiques selon la revendication 1, caractérisées en ce qu'elles contiennent comme émulsifiants des sels d'ammonium quaternaires tensioactifs.

**3.** Emulsions cosmétiques selon la revendication 2, caractérisées en ce qu'elles contiennent comme émulsifiants des sels d'ammonium quaternaires de formule

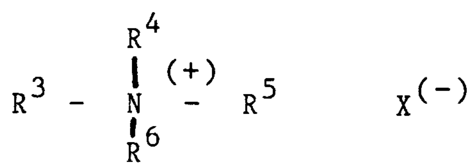

dans laquelle $R^3$ correspond à un groupement alkyle comportant 12 à 22 atomes de C, $R^4$ représente un groupement alkyle comprenant 1 à 4 atomes de C, un groupe benzyle ou un groupe $R^3$, $R^5$ et $R^6$ sont des groupements alkyle comportant 1 à 4 atomes de C et $X^{(-)}$ est un anion faisant partie du groupe chlorure, bromure et $R^7$-O-$SO_3^{(-)}$, dans laquelle $R^7$ équivaut à un groupement $C_1$-$C_4$.

**4.** Mise en oeuvre d'émulsions cosmétiques selon les revendications 1 à 3 pour les soins de la peau et/ou des cheveux.